# EUROPEAN PATENT SPECIFICATION

(11) **EP 4 211 126 B1**
(45) Date of publication and mention of the grant of the patent: **16.10.2024**
(21) Application number: 20862005.4
(22) Date of filing: 31.12.2020
(51) Int. Cl.: C07D 335/16, A61P 35/00, A61K 9/14

(54) **PHOTOCHEMICAL SYNTHESIS OF LOW PARTICLE SIZE MONODISPERSE GOLD NANOPARTICLES, LOADING WITH CANCER DRUG, AND DETERMINATION OF CYTOTOXIC ACTIVITY OF THE SAME**
PHOTOCHEMISCHE SYNTHESE VON MONODISPERSEN GOLDNANOPARTIKELN MIT GERINGER PARTIKELGRÖSSE, BELADUNG MIT KREBSMEDIKAMENT UND BESTIMMUNG DER ZYTOTOXISCHEN AKTIVITÄT DAVON
SYNTHÈSE PHOTOCHIMIQUE DE NANOPARTICULES D'OR MONODISPERSÉES À FAIBLE GRANULOMÉTRIE, CHARGEMENT AVEC UN MÉDICAMENT ANTICANCÉREUX ET DÉTERMINATION DE LEUR ACTIVITÉ CYTOTOXIQUE

(30) Priority: 14.09.2020 TR 202014545
(43) Date of publication of application: 19.07.2023
(73) Proprietor: Yildiz Teknik Universitesi, 34210 Istanbul (TR)
(72) Inventor: ARSU, Nergis, 34220 Esenler/Istanbul (TR); DIZMAN, Hüseyin Mirac, 41080 Kartepe/Kocaeli (TR); KURUCA, Dürdane Serap, 34093 Fatih/Istanbul (TR); ÖZEN EROGLU, Günes, 34520 Beylikdüzü/Istanbul (TR)
(74) Representative: Genç Ilhan, Oznur
(86) International application number: PCT/IB2020/001138
(87) International publication number: WO 2022/053845

(56) References cited:
- WO-A1-2017/116372
- WO-A2-2017/017063

## Description

### Technical Field of the Invention

The present invention relates to small-sized gold nanoparticles for medical use and a drug in which they are loaded with an active ingredient. The present invention is also directed to a chemical compound allowing the production of small-sized gold nanoparticles and a method of use thereof. The present invention also relates to a drug-loaded form of the nanoparticles conforming to this description and to a method for preparing same.

### Background Art

Doxorubicin (in short: DOXO) is one of the most used and working antitumor drugs in the treatment of cancer in recent years. Despite its effectiveness on cancer cells, the use thereof is limited due to its side effects such as cardiotoxicity and heart failure. Furthermore, it is known to cause some serious side effects such as renal, pulmonary, hematological, and testicular toxicity. Due to these side effects, nanoparticles have been used in recent years to load, deliver and release of drugs into tumorous tissues, because of their high surface-area-to-volume ratio [N. S. Elbialy, M. M. Fathy, and W. M. Khalil, "Doxorubicin loaded magnetic gold nanoparticles for in vivo targeted drug delivery," Int. J. Pharm., vol. 490, no. 1-2, pp. 190-199, 2015 (https://doi.org/10.1016/ j. ijpharm.2015.05.032)].

Thus, as in the case of doxorubicin as a cancer drug, it is possible to eliminate partially or completely said side effects by loading drugs having various side effects onto nanoparticles.

In addition, using the photochemical method for the synthesis of nanoparticles is gradually becoming an interesting method due to its low energy requirement and many other advantages such as simplicity, in addition to being fast and environmentally friendly [S. Yakut et al., "Dielectric properties of AuNPs/PEGMEA/PEGDA nanocomposite film prepared with an α-amino ketone by in-situ photochemical method," Phys. B Condens. Matter, vol. 542, pp. 6-11, 2018 (https://doi.org/10.1016/j.physb.2018.04.037); WO 2017/116372 A1].

There are many studies in the literature on the use of gold nanoparticles (in short: AuNps) in order to eliminate, particularly, the side effects of cancer drugs. In such studies, AuNps are usually synthesized using the simple reduction method, but the desired effect cannot be achieved because the AuNps are not functionalized. [R. K. Mohanty, S. Thennarasu, and A. B. Mandal, "Resveratrol stabilized gold nanoparticles enable surface loading of doxorubicin and anticancer activity," Colloids Surfaces B Biointerfaces, vol. 114, pp. 138-143, 2014 (https://doi.org/10.1016/ j.colsurfb.2013.09.057)]. Apart from this, in some studies, after the AuNps were synthesized, drug loading was performed by functionalizing them with various biocompatible molecules or polymers, and it is reported that the drug loaded onto AuNps was more effective on cancer cells, and its side effects were reduced. [J. Dennis Curry, Amanda Cameron, Bruce MacDonald, Collins Nganou, Hope Scheller, James Marsh, Stefanie Beale, Mingsheng Lu, Zhi Shan, Rajandra Kaliaperumal, Heping Xu, and X. Servos, Craig Bennett, Stephanie MacQuarrie, Ken D. Oakes, f, Martin Mkandawire, and Xu Zhang, "Adsorption of Doxorubicin on Citrate-Capped Gold Nanoparticles: Insights into Engineering Potent Chemotherapeutic Delivery Systems," Nanoscale, vol. 7, no. 46, 2015 (https://doi.org/10.1039/C5NR05826K)]. However, since the preparation of such nanoparticles usually requires more than one step, it results in loss of time and efficiency.

### Objects of the Invention

The main object of the invention is to provide solutions to the problems mentioned in the prior art.

Another object of the invention is to provide a cancer drug with minimized side effects of the active ingredient therein.

### Summary of the Invention

In this study, drug loading onto AuNps and release thereof is examined, which are photochemically prepared with a thioxanthone derivative photoinitiator in small-size and almost monodispersed form. Accordingly, the loading and release of the drug DOXO, which is one of the currently used cancer drugs, onto AuNps in a certain amount is examined (*for example, in terms of comparability with the results in Curry et al., 2015, supra*), and it is aimed to determine the toxic effect of the *selected reference* drug on cancer cells and eliminate its side effects.

In this study, monodisperse AuNps were synthesized using a single-step photochemical method using a thioxanthone derivative, which has a thioxanthone main structure known to be effective on cancer cells and has been used as a drug in the past years, but is not available in the literature yet, and it has been determined that the thioxanthone derivative used in the synthesis provides stability in size and dispersion of AuNps. In addition, with the advantage of the thioxanthone structure, it is found that DOXO, which is used as a cancer drug in the clinic, increases the activity of Au. As a result of all these effects, it is expected that the side effects caused by cancer drugs will be reduced.

### Brief Description of the Drawings

The drawings, whose brief description is provided below are just given for better understanding of the present invention and as such, are not intended to determine the scope of the claimed subject matter, in the absence of the description, wherein:

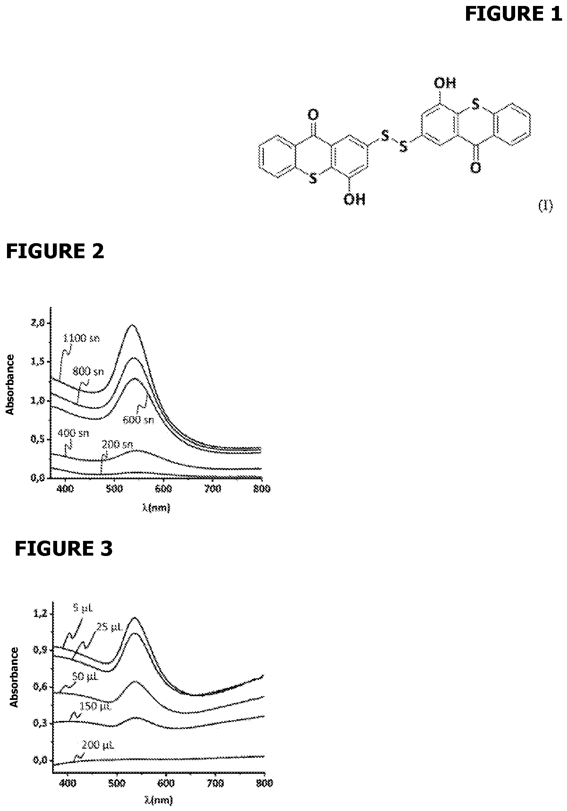

Figure 1 is an illustration of a compound [OH-TX-S-]₂ according to the present invention.
Figure 2 is a graph showing the progress of the formation of gold nanoparticles (AuNps) over time by the photochemical reduction method according to the present invention. Here, each curve from a curve where the peak at 535 nm (indicating the surface plasmon resonance band of the AuNps) is the flattest, to a curve where it is the sharpest, corresponds to the absorbance curves obtained in the photochemical reduction reaction at 200, 400, 600, 800 and 1100 seconds, respectively.
Figure 3 is a graph showing that the peak at 535 nm (showing the surface plasmon resonance band of the AuNps) becomes increasingly unobservable as the amount of DOXO solution added increases, starting from the curve corresponding to the end of 1100 seconds in Figure 2. The curves in the graph in question correspond from top to bottom, respectively, to the absorbance curves obtained when the volume of the DOXO solution added is 5, 25, 50, 150 and 200 microliters.

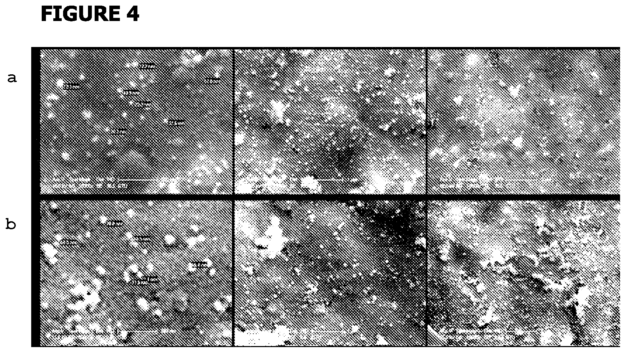

Figure 4 (a) is the SEM images where AuNps resulting from the photochemical reduction reaction appear. In the leftmost SEM image, the measurements of AuNps in the frame were taken, and the measurements (the particle diameters of the AuNps) were marked as 25.3, 30.5, 32.6, 37.2, 40.9, 52.0 and 73.1 nm. Exemplary/reference lengths in the lower parts of the SEM images from left to right are 500nm, 1 micrometer and 2 micrometers, respectively. Each SEM image was obtained with 15.0 kV voltage and 10.5 GTU.
Figure 4 (b) is the SEM images of AuNps coated with a cancer drug (here: DOXO). In the leftmost SEM image, the measurements of the coated AuNps in the frame were taken, and the measurements (the particle diameters of the coated AuNps) were marked as 60.1, 60.4, 66.5, 69.5, 71.0 and 78.0 nm. Exemplary/reference lengths in the lower parts of the SEM images from left to right are 500nm, 1 micrometer and 2 micrometers, respectively. Each SEM image was obtained with 15.0 kV voltage and 10.5 GTU.

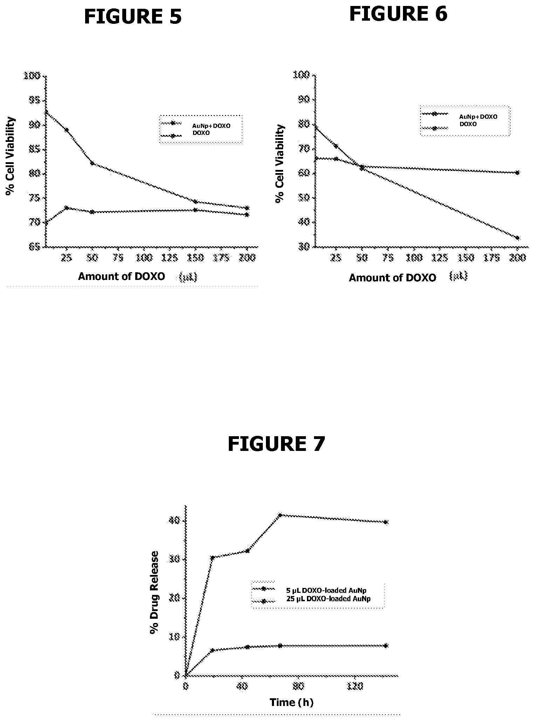

Figure 5 is a graph comparing the effect of a cancer drug alone (DOXO) and a cancer drug (AuNp+DOXO) coated on AuNps (prepared according to Example 2) on cell viability in cancer cells (here, acute promyelocytic leukemia, HL-60) in percent over time. The values on the abscess axis depict an amount of DOXO solution used in the exemplary study in microliters. The initial value where the abscess axis intersects the ordinate axis corresponds to the case in which DOXO solution was added in a volume of 5 microliters. Here, it is seen that the highest cytotoxic effect is achieved in DOXO-loaded AuNPs, which were produced in such a manner that DOXO solution was added at 5 microliters. It is observed that the cytotoxic effect does not vary significantly in AuNPs loaded with DOXO which were produced by adding DOXO solution in higher volumes. It is understood that DOXO in free form should be used in higher amounts in order to have a similar cytotoxic effect. Therefore, it is understood that the AuNPs loaded with the active ingredient (or drug, here: DOXO) of the present invention exhibit high cytotoxicity even when the drug is used in lower amounts, thus allowing to avoid side effects in effective manner. It is understood that the cytotoxic effect of the DOXO-loaded AuNPs of the present invention on HL-60 cells is very high.
Figure 6 is a graph comparing the effect of a cancer drug alone (DOXO) and a cancer drug (AuNp+DOXO) coated on AuNps (prepared according to Example 2) on cell viability in cancer cells (here, chronic myeloid leukemia, K562) in percent over time. The values on the abscess axis depict an amount of DOXO solution used in the exemplary study in microliters. The initial value where the abscess axis intersects the ordinate axis corresponds to the case in which DOXO solution was added in a volume of 5 microliters. The cytotoxic effect of the drug (here: DOXO) loaded AuNps does not appear to vary significantly with the amount of drug loaded.
Figure 7 is a graph comparing the drug release rates achieved with AuNPs loaded with different amounts of DOXO (prepared according to Example 3). Here, it is seen that the drug release rate of the DOXO-loaded AuNps achieved by adding 5 microliters of the DOXO solution is higher than the drug release rate of the DOXO-loaded AuNps achieved by adding 25 microliters of the DOXO solution.

### Detailed Description of the Invention

A compound [OH-TX-S-]₂ of Formula (I) and products obtained by using same in pharmaceutical production and relevant production methods are provided within the scope of the present invention. An illustration of the said compound is shown in Figure 1.

In this study, a new thioxanthone derivative, the compound 3,3'-disulfandiylbis (1-hydroxy-9H-thioxanthene-9-one) ([OH-TX-S-]₂), was used in the preparation of size-stable AuNps using a photochemical reduction method, which is an extremely simple, fast, environmentally friendly and efficient method. The dimensions of the synthesized AuNps were measured to be approximately 30 nm by DLS method, and DOXO, an effective cancer drug selected as a model drug, was loaded onto the AuNps in different amounts. In order to determine the efficacy of DOXO-loaded AuNps on cancer cells, it was examined using a MTT test, which is a standard cytotoxicity test.

When the compound [OH-TX-S-]₂, a thioxanthone derivative used within the scope of the invention, is used to obtain AuNps by reducing a salt of Au, it provides a higher size stability than the other known thioxanthone derivatives and increases the controllability of the AuNp particle sizes. Thus, it makes the AuNps obtained acceptable as monodispersed (improves the polydispersity index, referred to as PdI for short).

Other than 30 nm, larger sizes of AuNps, e.g., 60 nm will also work; however, it cannot be expected to perform as much as the smaller-sized AuNps, e.g., 30 nm. In fact, since a small particle size of AuNps increases the surface area, the loading performance, and the release rate increase.

### EXAMPLE 1:

The synthesis of the compound [OH-TX-S-]₂ can be carried out as follows, in order to ensure the size stability of AuNps and to be used as a photoreducing agent that has a primary role in photoreduction of the HAuCl₄ salt:
- Once H₂SO₄ (e.g., 7.0 mL) was added on thiosalicylic acid (e.g., 4×10⁻³ moles) and stirred in a flask (e.g., for 5-10 min), 3,3-dihydroxy phenyl disulfide (e.g., 1×10⁻³ moles) dissolved in benzene (e.g., 10 mL) was added dropwise and refluxed (e.g., for 1 hour at room temperature, followed by e.g., 12 hours at 80°C).
- Then, (after the reaction mixture was left to stand e.g., at room temperature, e.g., overnight), the reaction was terminated (by pouring the reaction mixture into e.g., about 200 mL of hot distilled water). The product precipitated in the water was filtered off and the solid was dried in a vacuum oven.
- After having been washed (e.g., with petroleum ether), the dried solid was purified (e.g., in a column system, in the presence of ethanol: ethyl acetate mobile phase (e.g., in a ratio of 2:1.5 by volume)), thereby obtaining pure [OH-TX-S-]₂.

### EXAMPLE 2:

The compound [OH-TX-S-]₂ obtained in pure form in Example 1 was used for the synthesis of stable AuNps by the photochemical method. Thiyl radicals are formed from the disulfide of the said compound when it is illuminated by a lamp of the appropriate wavelength (e.g., 365 nm). With these radicals, Au⁺³ ions are reduced and Au° is obtained first. Subsequently, AuNps start to form when metallic gold induces a nucleation reaction depending on the illumination. AuNPs are surrounded by [OH-TX-S-]₂ as a result of a gold-thiol interaction, and stable AuNPs are obtained. The following steps can be applied sequentially to synthesize AuNps by the photoreduction process;
- First, (e.g., 0.5 mg (9.5×10⁻⁴ mmoles)) [OH-TX-S-]₂ and (e.g., 34 mg (9.5×10⁻² mmoles)) HAuCl₄ were dissolved (in a co-solvent, e.g., 10 mL DMSO), then diluted with water (e.g., to a water:DMSO ratio of 99:1 by volume) (e.g., at final concentrations of [3.75×10⁻³ mM] and [3.75×10⁻¹ mM], respectively). Thus, a "first mixture" in solution was obtained. The purpose of the process of "dilution with water" in this step is to ensure that the lethal effect of the targeted drug-loaded AuNps on cancer cells is not masked by the possible lethal effect of the co-solvent (here, DMSO) used to dissolve [OH-TX-S-]₂ and the gold salt, so that it can be observed accurately.
- The first mixture (e.g., with a water:DMSO ratio of 99:1 by volume) was photochemically reduced (e.g., with 365 nm, medium-pressure mercury lamp, (HAMAMATSU LIGHTNINGCURE Spot light source LC8)) and the formation of AuNps (e.g., with UV-Vis spectrophotometer, as a function of time) was followed. At the end of 300 seconds, it was observed that the surface plasmon resonance band of AuNps at 535 nm began to appear (Figure 2).
- It was observed that the color of the solution changed from light yellow to pink, the characteristic color of AuNps, depending on the lighting time. Based on these observations, a series of photographs was obtained showing that the medium/mixture, in which the photochemical reduction reaction was carried out, gradually became colored though it was initially colorless. The photographs in the series correspond to the optical images obtained in the initial (zero second), 400, 600, 800 and 1100 second progress in the photochemical reduction reaction, respectively. Here, the progress in the change of optical properties (darkening/reddening of the reaction medium) shows that the gold salt is converted into gold nanoparticles.
- As a result of this process step, a "first suspension" containing AuNps was obtained.

### EXAMPLE 3:

DOXO [1×10⁻⁴ M] from 5 µL to 200 µL was added to the AuNps formed after the illumination for 1100 seconds. With this process step:
- After each addition of DOXO, the solution was mixed (e.g., for 15 minutes) to fully homogenize it, while at the same time, it was ensured that DOXOs adhere onto the surface of the AuNps. The change in the color of the AuNp solution depending on the amount of DOXO loading was observed through a series of photographs. During the observation, the solution, which was pink before the addition of DOXO, turned purple after the addition of 100 µL of DOXO, since agglomeration occurred between AuNps as a result of non-covalent interactions (hydrogen bonds, pi-pi interactions, etc.) between DOXO adsorbed by AuNps. When the amount of DOXO added was increased to 200 µL, the purple color thereof disappeared completely, and the solution became colorless. The series of photographs accompanying these observations show that by coating the gold nanoparticles with a cancer drug active ingredient (here: DOXO), their visibility gradually decreases, resulting in a gradual change in appearance from red to colorless. The photographs in the series correspond to the optical images where the volume of the DOXO solution added is 0, 5, 50, 100 and 200 microliters, respectively. When the volume of the DOXO solution added reached 200 microliters, it was observed that the optical properties of the medium turned from red to completely colorless.
- After each addition, the change in SPR of the AuNps was followed by UV-Vis spectrophotometer. After adding 200 µL of DOXO, it was observed that the SPR of the AuNps were completely extinguished (Figure 3). Table 1 shows the DLS results of AuNps without DOXO loading and AuNps with various concentrations of DOXO loaded. According to these results, it is apparent that AuNps prepared using [OH-TX-S-]₂ have expediently very small sizes such as 30 nm and dimensional stability. In addition, depending on the amount of DOXO loading, the size of AuNps increases, which is clearly resulting from non-covalent interactions between doxorubicins loaded onto AuNps, as mentioned above. The SEM analysis also supports the DLS results. As seen from Figure 4, while the size of the AuNPs was around 30 nm before the addition of DOXO (a) it was observed that the size thereof reached 60-70 nm after addition of 5 µL of DOXO (b).

**Table 1. The average particle diameters of the coated AuNps achieved based on the amount of DOXO solution added in Example 3 and the corresponding polydispersity index values (the diameters and PdI values of the AuNp cores obtained by Example 2 before the DOXO solution was added, are shown in the column where the added amount of DOXO solution is "0").**

| | **The amount of DOXO solution (µL) containing DOXO at a concentration of 1×10⁻⁴ mol/L added onto the "first suspension"** | | | | | |
|---|---|---|---|---|---|---|
| | ***0*** | ***5*** | ***25*** | ***50*** | ***150*** | ***200*** |
| **AuNp diameters: Z-Ave (nm) *(including coating, if any)*** | 29.46 | 36.33 | 40.29 | 108.5 | 384.4 | 280.3 |
| | 30.44 | 37.37 | 38.06 | 121.8 | 298.3 | 196.6 |
| | 30.41 | 37.53 | 37.38 | 127.3 | 379.0 | 160.9 |
| **PdI** | 0.446 | 0.447 | 0.447 | 0.266 | 0.418 | 0.335 |
| | 0.451 | 0.455 | 0.482 | 0.248 | 0.331 | 0.202 |
| | 0.450 | 0.470 | 0.412 | 0.221 | 0.483 | 0.169 |

Accordingly, with the present invention, gold nanoparticles (AuNps) with a gold core coated with a coating layer containing DOXO (in varying amounts) are obtained; and it was ensured that the diameter of the gold core is in the range of 30±1 nm with a PdI of 0.45±0.01. In this scope, it is ensured that:
- the diameter of AuNps is in the range of 37±1 nm with a PdI of 0.447 to 0.470 when 5 microliters of DOXO is loaded on the gold core;
- the diameter of AuNps is in the range of 37.38 to 40.29 nm with a PdI of 0.412 to 0.447 when 25 microliters of DOXO is loaded on the gold core; and
- the diameter of AuNps is in the range of 108.5 to 127.3 nm with a PdI of 0.221 to 0.266 when 50 microliters of DOXO is loaded on the gold core.

### EXAMPLE 4:

In order to determine the amount of DOXO loaded on the AuNps, calculations were performed by using DOXO at different concentrations, and plotting a calibration line with the fluorescence emission intensities. The amount of the free DOXO was checked by measuring the fluorescence emission intensities after each addition of DOXO onto the AuNps. The value calculated was subtracted from the initial amount added, and the amount of DOXO adsorbed on the surface of the AuNps by non-covalent interaction was determined. As a result of the calculations, the (maximum) amount of DOXO adsorbed in Example 3 was found to be 10.67 µg (λ_{uy} = 480 nm was chosen for DOXO in fluorescence measurements). As a result, it was determined that only 0.2 µg of the added 10.87 µg DOXO was not adsorbed.

### EXAMPLE 5:

MTT (3- (4,5-dimethylthiazol-2-yl) -2,5-diphenyl-2H-tetrazolium bromide) colorimetric test allows the quantitation of living cells whose mitochondrial activity is maintained in the culture medium. The MTT colorimetric test was performed according to the following procedure:
- According to the standard procedure under cell culture conditions, cells were maintained in RPMI-1640 culture medium containing 10% FBS and 1% penicillin/streptomycin.
- The trypan blue test was used for cell counts.
- For the evaluation of cytotoxicity, the cells were cultured in 96-well flat bottom plates and 10 µl of sample and 90 µl of cell suspension were applied at 10,000 cells/100 µL complete medium per well.
- Cell-cultivated plates were taken to an incubator and incubated for 72 hours at 37°C in 5% CO2 environment.
- At the end of the period, 10 µL of MTT solution (in 5 mg/mL in PBS) was added to each well and allowed to incubate for 3.5 hours.
- After checking the formazan crystals under the microscope, 50 µl of the liquid in the wells was removed.
- The crystals formed were dissolved by adding 200 µl DMSO in wells.
- The optical density, which shows the colorimetric change in the wells, was measured in a microplate reader at a wavelength of 560 nm at a reference wavelength of 620 nm.
- The absorbance values obtained were compared to the control, and the percent-viability data were generated and evaluated statistically by using Graphpad Prism 5.04 software with an unpaired t test. P<0.05 was considered significant.

According to MTT experiments, after 72 hours of incubation, the effect of DOXO in free form, i.e., not loaded on AuNps, as well as the DOXO-loaded AuNps was investigated for acute promyelocytic leukemia (HL-60) and chronic myeloid leukemia (K562) cells, and the results are given in Figure 5 and Figure 6, respectively.

When examined for the K562 cells, as seen in Figure 5; it was determined that AuNPs loaded with 5, 25 and 50 µL DOXO are more effective than DOXO alone.

It was determined that the same applies to HL-60 cells, and AuNPs loaded with 5 and 25 µL DOXO showed a higher cytotoxic effect than that of DOXO alone which was administered in the same amount (Figure 6).

For the investigation of drug release, AuNps loaded with 5 and 25 µL DOXO, which were apparently the most effective formulations, were selected. For release, DOXO loaded solutions were added into a phosphate buffer (pH=7.3), and then agitated at 37°C at 200 RPM for about 140 hours. Fluorescence measurements of the samples taken at regular intervals were carried out and the amount of DOXO released was calculated through the calibration curve. At the end of the first 67 hours, the release rate was found to be 40% in the sample loaded with 5 µL, while the release rate was calculated as 7% in the sample loaded with 25 µL. When the duration was extended to 140 hours, it was observed that the amount of release of the two samples did not change significantly as compared to 67 hours (Figure 7).

**Table 2. The compositions used in the examples**

| | | | |
|---|---|---|---|
| | From EXAMPLE 2: Initial solution in the Au reduction step (Mixture 1) | From EXAMPLE 3: Following the reduction of Au in the initial solution; the amount of DOXO solution added to replace the photoinitiator on the surface of the reduced Au (in this case AuNp) (to coat the AuNps with the drug) | The total volume from the operations in EXAMPLE 3 (Mixture 2: AuNp-containing suspension) |

| Sample #: | HAuCl₄ (3.75×10⁻¹ mM) + [OH-TX-S-]₂ (3.75×10⁻³ mM) | Addition of DOXO (1.0×10⁻¹ mM) | Total Amount |
|---|---|---|---|
| 0 | 2.7 mL | - | 2.700 mL |
| 1*^{,}** | 2.7 mL | 5 µL | 2.705 mL |
| 2*^{,}** | 2.7 mL | 25 µL | 2.725 mL |
| 3* | 2.7 mL | 50 µL | 2,750 mL |
| 4 | 2.7 mL | 100 µL | 2,800 mL |
| 5 | 2.7 mL | 150 µL | 2.850 mL |
| 6 | 2.7 mL | 200 µL | 2.900 mL |

| | | | |
|---|---|---|---|
| **) preferred addition amounts of the solution containing the active ingredient (or drug, here: DOXO)* ***) more preferred addition amounts of the solution containing the active ingredient (or drug, here: DOXO)* | | | |

The preferred concentration of [OH-TX-S-]₂, which is most suitable for obtaining monodisperse and size-stable AuNps, is about 1/100 of the Au salt concentration (a range of 1/80 to 1/120 can be suggested). As the Au salt concentration range in which this approach is considered to be valid, 3.50×10⁻⁶ mol/liter to 4.00×10⁻⁶ mol/liter can be suggested.

In order for the gold peak to be invisible (i.e., to ensure that the AuNp is completely coated), it was sufficient to add 200 microliters (0.2×10⁻³ liters) of 1.0×10⁻⁴ mol/liter DOXO solution on 2700 microliters (2,7×10⁻³ liters) of 3,75×10⁻⁶ mol/liter Au salt solution. From Figure 5 and Figure 6; the fact that a satisfactory cytotoxicity can be achieved when the DOXO solution is added at 5 microliters, 25 microliters and 50 microliters is interpreted such that the compound [OH-TX-S-]₂ contained in the coating layer on the AuNps also has cytotoxicity effect (especially on HL-60) in such cases.

From the Table 2, the molar concentrations that can be preferred in the mixture with the active ingredient (or drug, here: DOXO) added, can be calculated as follows:
- In the case corresponding to the addition of 5 to 200 microliters of DOXO solution, the Au concentration of the second suspension may be in the following range:
   (3.75×10⁻⁶ mol/liter) × 2.7/2.9 = 3.49 × 10⁻⁶ mol/liter (approximately, or preferably precisely) to (3.75×10⁻⁶ mol/liter) × 2.7/2.705 = 3.74 × 10⁻⁶ mol/liter (approximately, or preferably precisely).
- In the case corresponding to the addition of 5 to 50 microliters of DOXO solution, the Au concentration of the second suspension may be in the following range:
   (3.75×10⁻⁶ mol/liter) × 2.7/2.750 = 3.68x 10⁻⁶ mol/liter (approximately, or preferably precisely) to (3.75×10⁻⁶ mol/liter) × 2.7/2.705 = 3.74 × 10⁻⁶ mol/liter (approximately, or preferably precisely).
- In the case corresponding to the addition of 5 to 25 microliters of DOXO solution, the Au concentration of the second suspension may be in the following range:
   (3.75×10⁻⁶ mol/liter) × 2.7/2.725 = 3.68x 10⁻⁶ mol/liter (approximately, or preferably precisely) to (3.75×10⁻⁶ mol/liter) × 2.7/2.705 = 3.72 × 10⁻⁶ mol/liter (approximately, or preferably precisely).
- In the case corresponding to the addition of 5 to 200 microliters of DOXO solution, the DOXO concentration of the second suspension may be in the following range:
   (1.0×10⁻⁴ mol/liter) × 0.005/2.705 = 1.85 × 10⁻⁹ mol/liter (approximately, or preferably precisely) to (1.0×10⁻⁴ mol/liter) × 0.2/2,9 = 6.90 × 10⁻⁶ mol/liter (approximately, or preferably exactly).
- In the case corresponding to the addition of 5 to 50 microliters of DOXO solution, the DOXO concentration of the second suspension may be in the following range:
   (1.0×10⁻⁴ mol/liter) × 0.005/2.705 = 1.85 × 10⁻⁹ mol/liter (approximately, or preferably precisely) to (1.0×10⁻⁴ mol/liter) × 0.050/2.750 = 1.82 × 10⁻⁸ mol/liter (approximately, or preferably precisely).
- In the case corresponding to the addition of 5 to 25 microliters of DOXO solution, the DOXO concentration of the second suspension may be in the following range:
   (1.0×10⁻⁴ mol/liter) × 0.005/2.705 = 1.85 × 10⁻⁹ mol/liter (approximately, or preferably precisely) to (1.0×10⁻⁴ mol/liter) × 0.025/2.725 = 9.17 × 10⁻⁷ mol/liter (approximately, or preferably precisely).

With the study demonstrating the present invention:
- The synthesis of a photoreducing agent that can provide the size and dispersion stability of the AuNps prepared photochemically is performed: the photoreducing agent [OH-TX-S-]₂ synthesized and used in this study was used for the first time in the synthesis of AuNps;
- The size of AuNps synthesized in solution in the presence of photoinitiator is around 30 nm, which enables the use of the AuNps in the drug delivery system.
- It has been evaluated that the -S-S- bonds and/or thiyl radicals in the structure of [OH-TX-S-]₂ performing the photoreduction process, allow the dimensional stabilization of AuNps.
- DOXOs selected as model cancer drugs were loaded onto AuNps at different proportions and the loaded amounts were calculated by spectrophotometric methods.
- Their activities in acute and chronic leukemia cells were examined by MTT method for AuNps and AuNps loaded with DOXO, respectively, and it was determined that AuNps containing low concentrations of DOXO were particularly effective on cancer cells.
- Additionally, it has been observed that AuNPs with a coating layer containing the compound [OH-TX-S-]₂ of Formula (I) have a high cytotoxic effect on cancer cells. Especially, due to the effect of the said compound according to the Formula (I) allowing to have a reduced diameter of the gold core, it is ensured that the particle diameter of the coated AuNps is small and thus their surface/volume ratio is large. In this way, it is possible for particles containing either DOXO, or the compound [OH-TX-S-]₂ of Formula (I), or both of them to exhibit a high cytotoxicity on cancer cells. The invention, which allows the active ingredient to be delivered to the body in lower amounts by loading the active ingredient onto the AuNps having a low particle size, has thus made it possible to avoid side effects.
- Drug release times from AuNps were examined, allowing a comparison on efficiency.
- It has been found that another important reason for the effectiveness of the photoinitiator, which was synthesized for the first time and used in the preparation of AuNps, is that some derivatives thereof containing a thioxanthone unit in its structure was previously used in the treatment of leukemia, and the contribution resulting from the fact that AuNps prepared in the presence of this photoinitiator are surrounded by [OH-TX-S-]₂ cannot be ignored.

In this study, a positive effect resulting from the successful loading, and release, of a cancer drug selected as a model onto AuNPs, which are synthesized to be monodispersed and 30 nm in size by a highly efficient and inexpensive method so as to be used in the release of a cancer or another drug (neurological, etc.), shows that the carrier AuNps that increase the efficacy of the drugs can be commercially synthesized and it is quite likely to use them in the pharmaceutical industry.

In view of the above:
A compound [OH-TX-S-]₂ of Formula (I) is provided within the scope of the present invention. There are also provided gold nanoparticles (AuNp) coated with a coating layer containing the compound [OH-TX-S-]₂ of Formula (I) within the scope of the present invention.

In addition, within the scope of the present invention, gold nanoparticles (AuNps) with a gold core coated with a coating layer containing a cancer drug (e.g., DOXO) are provided; and for the said AuNps, it is possible to have a diameter of the gold core of 30±1 nm, with a PdI of 0.45±0.01. In the said AuNps, it is possible to have a diameter of the coated AuNps in the range of 36.33 to 384.4 nm, with a PdI in the range of 0.169 to 0.482. In a preferred embodiment of the invention, for the said AuNps, it is possible to have a diameter of the coated AuNps in the range of 36.33 to 127.3 nm, with a PdI in the range of 0.221 to 0.482 (this corresponds to the addition of 5 to 50 microliters of DOXO solution in the examples). In another preferred embodiment of the invention, for the said AuNps, it is possible to have a diameter of the coated AuNps in the range of 36.33 to 40.29 nm, with a PdI in the range of 0.412 to 0.482 (this corresponds to the addition of 5 to 25 microliters of DOXO solution in the examples).

In addition, within the scope of the present invention, a method for the production of the compound [OH-TX-S-]₂ of Formula (I) is provided, the method comprising the following steps sequentially:
i) obtaining a thiosalicylic acid - H₂SO₄ mixture by adding H₂SO₄ onto thiosalicylic acid and mixing same;
ii) adding dropwise a solution of 3,3-dihydroxy phenyl disulfide obtained by dissolving 3,3-dihydroxy phenyl disulfide in benzene, onto said thiosalicylic acid - H₂SO₄ mixture; thereby obtaining a mixture comprising thiosalicylic acid, H₂SO₄ and 3,3-dihydroxy phenyl disulfide;
iii) refluxing the mixture comprising thiosalicylic acid, H₂SO₄ and 3,3-dihydroxy phenyl disulfide;
iv) performing a reaction in the said refluxed mixture comprising thiosalicylic acid, H₂SO₄ and 3,3-dihydroxy phenyl disulfide;
v) terminating the reaction in step (iv), resulting in a product mixture;
vi) filtering, drying, washing, and purifying the product mixture obtained in step (v).

Said method may comprise one or more of the following:
m) having a concentration of the thiosalicylic acid in the thiosalicylic acid - H₂SO₄ mixture obtained in step (i) being in the range of 3.5×10⁻³ moles to 4.5×10⁻³ moles per 7 mL of H₂SO₄;
n) having a concentration of 3,3-dihydroxy phenyl disulfide in the solution of 3,3-dihydroxy phenyl disulfide obtained by dissolving 3,3-dihydroxy phenyl disulfide in benzene in step (ii) being in the range of 0.75×10⁻³ moles to 1.25×10⁻³ moles per 10 mL of benzene;
o) applying the reflux process in step (iii) for at least 30 minutes at room temperature followed by at least 6 hours at a temperature in the range 75-85°C;
p) performing the termination of the reaction in step (v) by precipitation of the refluxed mixture comprising thiosalicylic acid, H₂SO₄ and 3,3-dihydroxy phenyl disulfide, which was reacted in step (iv), in hot water;
r) performing the drying process of step (vi) in a vacuum oven;
s) performing the washing process of step (vi) using petroleum ether;
t) conducting the purification in step (vi) in the presence of an ethanol:ethyl acetate mobile phase, using a column system.

In addition, within the scope of the present invention, the use of the compound [OH-TX-S-]₂ of Formula (I) in the production of gold nanoparticles (AuNps) by photochemical reduction is proposed, and said method of use comprises the following steps:
a) preparing a first mixture containing the compound [OH-TX-S-]₂ in solution together with a gold salt;
b) obtaining a first suspension containing AuNps by subjecting the first mixture to a photochemical reduction process.

In a possible variation of the said method of use; the first mixture may contain, as a gold salt, HAuCl₄ dissolved in a cosolvent and then diluted with water to a concentration of 3 × 10⁻⁶ to 4 × 10⁻⁶ mol/liter, as well as [OH-TX-S-]₂ at a concentration range corresponding to 1/80 to 1/120 of the said concentration.

In the said method of use, the photochemical reduction process can be carried out by subjecting the first mixture to a radiation corresponding to that provided by a medium-pressure mercury lamp at 365 nm for more than 300 seconds; thereby allowing the appearance of the AuNp surface plasmon resonance band at 535 nm.

Said method of use may include a step (c) of producing a second suspension by mixing the first suspension with a solution of a cancer drug, following step (b).

Said cancer drug (active ingredient) may be DOXO. In this case, the concentration of DOXO in the second suspension may be in the range of 1.85 × 10⁻⁹ mol/liter to 9.17 × 10⁻⁷ mol/liter (this corresponds to the addition of 5 to 25 microliters of DOXO solution in the examples). The concentration of DOXO in the second suspension may be in the range of 1.85 × 10⁻⁹ mol/liter to 1.82 × 10⁻⁸ mol/liter (this corresponds to the addition of 5 to 50 microliters of DOXO solution in the examples).

## Claims

1. A compound [OH-TX-S-]₂ of Formula (I):

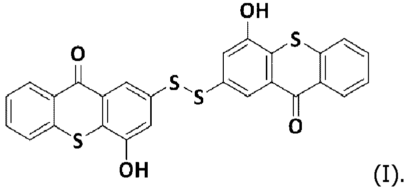

2. Gold nanoparticles (AuNps) coated with a coating layer containing the compound [OH-TX-S-]₂ of Formula (I)

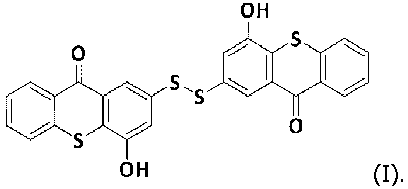

3. Gold nanoparticles (AuNps) according to claim 2, wherein the gold nanoparticles (AuNps) have a gold core coated with a coating layer containing doxorubicin and the diameter of the gold core is 30±1 nm with a PdI of 0.45±0.01.

4. Method for the production of the compound [OH-TX-S-]₂ of formula (I), comprising the following steps in sequence:
i) obtaining a thiosalicylic acid - H₂SO₄ mixture by adding H₂SO₄ onto thiosalicylic acid and mixing same;
ii) adding dropwise a solution of 3,3-dihydroxy phenyl disulfide obtained by dissolving 3,3-dihydroxy phenyl disulfide in benzene, onto said thiosalicylic acid - H₂SO₄ mixture; thereby obtaining a mixture comprising thiosalicylic acid, H₂SO₄ and 3,3-dihydroxy phenyl disulfide;
iii) refluxing the mixture comprising thiosalicylic acid, H₂SO₄ and 3,3-dihydroxy phenyl disulfide;
iv) performing a reaction in the said refluxed mixture comprising thiosalicylic acid, H₂SO₄ and 3,3-dihydroxy phenyl disulfide;
v) terminating the reaction in step (iv), resulting in a product mixture;
vi) filtering, drying, washing, and purifying the product mixture obtained in step (v)

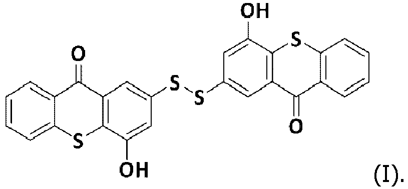

5. The method according to claim 4, comprising one or more of the following:
m) having a concentration of the thiosalicylic acid in the thiosalicylic acid - H₂SO₄ mixture obtained in step (i) being in the range of 3.5×10⁻³ moles to 4.5×10⁻³ moles per 7 mL of H₂SO₄;
n) having a concentration of 3,3-dihydroxy phenyl disulfide in the solution of 3,3-dihydroxy phenyl disulfide obtained by dissolving 3,3-dihydroxy phenyl disulfide in benzene in step (ii) being in the range of 0.75×10⁻³ moles to 1.25×10⁻³ moles per 10 mL of benzene;
o) applying the reflux process in step (iii) for at least 30 minutes at room temperature followed by at least 6 hours at a temperature in the range 75-85°C;
p) performing the termination of the reaction in step (v) by precipitation of the refluxed mixture comprising thiosalicylic acid, H₂SO₄ and 3,3-dihydroxy phenyl disulfide, which was reacted in step (iv), in hot water;
r) performing the drying process of step (vi) in a vacuum oven;
s) performing the washing process of step (vi) using petroleum ether;
t) conducting the purification in step (vi) in the presence of an ethanol:ethyl acetate mobile phase, using a column system.

6. A method for the use of the compound [OH-TX-S-]₂ of Formula (I) in the production of gold nanoparticles (AuNps) by photochemical reduction, comprising the following steps:
a) preparing a first mixture containing the compound [OH-TX-S-]₂ in solution together with a gold salt;
b) obtaining a first suspension containing AuNps by subjecting the first mixture to a photochemical reduction process

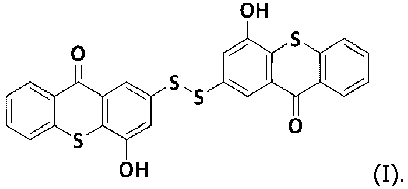

7. The method according to claim 6, wherein the first mixture contains, as a gold salt, HAuCl₄ dissolved in a cosolvent and then diluted with water to a concentration of 3 × 10⁻⁶ to 4 × 10⁻⁶ mol/liter, as well as [OH-TX-S-]₂ at a concentration range corresponding to 1/80 to 1/120 of the said concentration.

8. The method according to any one of claim 6 or 7, wherein the photochemical reduction process is carried out by subjecting the first mixture to a radiation corresponding to that provided by a medium-pressure mercury lamp at 365 nm for more than 300 seconds.

9. The method according to any one of claims 6 to 8, comprising a step (c) of producing a second suspension by mixing the first suspension with a solution of a cancer drug, following step (b).

10. The method according to claim 8, wherein said cancer drug is doxorubicin.

11. The method according to claim 10, wherein the concentration of doxorubicin in the second suspension is in the range of 1.85 × 10⁻⁹ mol/liter to 9.17 × 10⁻⁷ mol/liter.

12. The method according to claim 11, wherein the concentration of doxorubicin in the second suspension is in the range of 1.85 × 10⁻⁹ mol/liter to 1.82 × 10⁻⁸ mol/liter.

## Patentansprüche

1. Verbindung [OH-TX-S-]₂ der Formel (I):

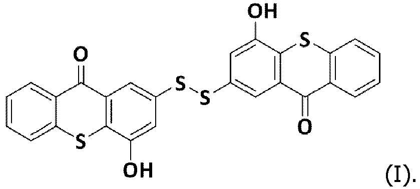

2. Goldnanopartikel (AuNps), beschichtet mit einer Überzugsschicht, die die Verbindung [OH-TX-S-]₂ der Formel (I) enthält

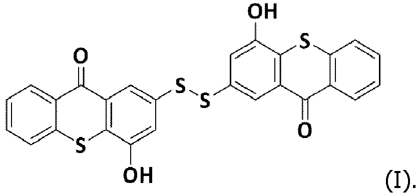

3. Goldnanopartikel (AuNps)nach Anspruch 2, wobei die Goldnanopartikel (AuNps) einen Goldkern aufweisen, der mit einer Doxorubicin enthaltenden Überzugsschicht beschichtet ist, und der Durchmesser des Goldkerns 30±1 nm mit einem PdI von 0,45±0,01 beträgt.

4. Verfahren zur Herstellung der Verbindung [OH-TX-S-]₂ der Formel (I), das nacheinander die folgenden Schritte umfasst:
i) Herstellung einer Mischung aus Thiosalicylsäure und H₂ SO₄ durch Zugabe von H₂ SO₄ zu Thiosalicylsäure und Mischen derselben;
ii) tropfenweise Zugabe einer Lösung von 3,3-Dihydroxyphenyldisulfid, die durch Lösen von 3,3-Dihydroxyphenyldisulfid in Benzol erhalten wurde, zu dem Gemisch aus Thiosalicylsäure, H₂ SO₄ ; dadurch erhält man ein Gemisch, das Thiosalicylsäure, H₂ SO₄ und 3,3-Dihydroxyphenyldisulfid enthält;
iii) Rückfluss der Mischung aus Thiosalicylsäure, H₂ SO₄ und 3,3-Dihydroxyphenyldisulfid;
iv) Durchführung einer Reaktion in der genannten refluxierten Mischung, die Thiosalicylsäure, H₂ SO₄ und 3,3-Dihydroxyphenyldisulfid enthält;
v) Beenden der Reaktion in Schritt (iv), wobei ein Produktgemisch entsteht;
vi) Filtrieren, Trocknen, Waschen und Reinigen des in Schritt (v) erhaltenen Produktgemischs

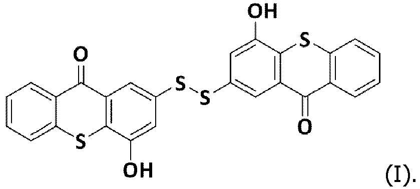

5. Verfahren nach Anspruch 4, umfassend eine oder mehrere der folgenden Maßnahmen:
m) mit einer Konzentration der Thiosalicylsäure in der in Schritt (i) erhaltenen Thiosalicylsäure-H₂ SO₄ -Mischung im Bereich von 3,5×10⁻³ -Molen bis 4,5×10⁻³ - Molen pro 7 mL H₂ SO₄;
n) mit einer Konzentration von 3,3-Dihydroxyphenyldisulfid in der Lösung von 3,3-Dihydroxyphenyldisulfid, die durch Lösen von 3,3-Dihydroxyphenyldisulfid in Benzol in Schritt (ii) erhalten wurde, im Bereich von 0,75×10⁻³ Molen bis 1,25×10⁻³ Molen pro 10 mL Benzol;
o) Anwendung des Rückflussverfahrens in Schritt (iii) für mindestens 30 Minuten bei Raumtemperatur, gefolgt von mindestens 6 Stunden bei einer Temperatur im Bereich von 75-85°C;
p) die Beendigung der Reaktion in Schritt (v) durch Ausfällen der in Schritt (iv) umgesetzten, unter Rückfluss erhaltenen Mischung aus Thiosalicylsäure, H₂SO₄ und 3,3-Dihydroxyphenyldisulfid in heißem Wasser;
r) Durchführung des Trocknungsprozesses von Schritt (vi) in einem Vakuumofen;
s) Durchführung des Waschverfahrens von Schritt (vi) mit Petrolether;
t) Durchführung der Reinigung in Schritt (vi) in Anwesenheit einer mobilen Phase Ethanol:Ethylacetat unter Verwendung eines Säulensystems.

6. Verfahren zur Verwendung der Verbindung [OH-TX-S-]₂ der Formel (I) bei der Herstellung von Gold-Nanopartikeln (AuNps) durch photochemische Reduktion, umfassend die folgenden Schritte:
a) Herstellung einer ersten Mischung, die die Verbindung [OH-TX-S-]₂ in Lösung zusammen mit einem Goldsalz enthält;
b) Gewinnung einer ersten Suspension, die AuNps enthält, indem die erste Mischung einem photochemischen Reduktionsprozess unterzogen wird

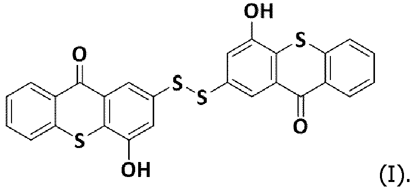

7. Verfahren nach Anspruch 6, wobei die erste Mischung als Goldsalz HAuCl₄, das in einem Co-Lösungsmittel gelöst und dann mit Wasser auf eine Konzentration von 3 × 10⁻⁶ bis 4 × 10⁻⁶ mol/Liter verdünnt wurde, sowie [OH-TX-S-]₂ in einem Konzentrationsbereich enthält, der 1/80 bis 1/120 der genannten Konzentration entspricht.

8. Verfahren nach einem der Ansprüche 6 oder 7, wobei der photochemische Reduktionsprozess durchgeführt wird, indem das erste Gemisch mehr als 300 Sekunden lang einer Strahlung ausgesetzt wird, die derjenigen einer Mitteldruck-Quecksilberlampe bei 365 nm entspricht.

9. Verfahren nach einem der Ansprüche 6 bis 8, umfassend einen Schritt (c) der Herstellung einer zweiten Suspension durch Mischen der ersten Suspension mit einer Lösung eines Krebsmedikaments im Anschluss an Schritt (b).

10. Verfahren nach Anspruchs, wobei das Krebsmedikament Doxorubicin ist.

11. Verfahren nach Anspruch 10, wobei die Konzentration von Doxorubicin in der zweiten Suspension im Bereich von 1,85 × 10⁻⁹ mol/Liter bis 9,17 × 10⁻⁷ mol/Liter liegt.

12. Verfahren nach Anspruch 11, wobei die Konzentration von Doxorubicin in der zweiten Suspension im Bereich von 1,85 × 10⁻⁹ mol/Liter bis 1,82 × 10⁻⁸ mol/Liter liegt.

## Revendications

1. - Composé [OH-TX-S-]₂ de Formule (I) :

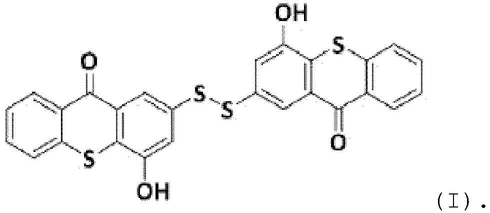

2. - Nanoparticules d'or (AuNps) revêtues d'une couche de revêtement contenant le composé [OH-TX-S-]₂ de Formule (I) :

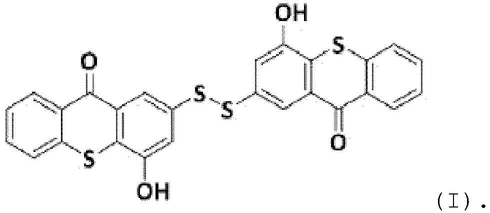

3. - Nanoparticules d'or (AuNps) selon la revendication 2, dans lesquelles les nanoparticules d'or (AuNps) ont un coeur d'or revêtu d'une couche de revêtement contenant de la doxorubicine et le diamètre du coeur d'or est de 30 ± 1 nm avec un indice de polydispersité (PdI) de 0,45 ± 0,01.

4. - Procédé de production du composé [OH-TX-S-]₂ de formule (I), comprenant les étapes suivantes dans l'ordre :
i) obtenir un mélange acide thiosalicylique - H₂SO₄ par addition de H₂SO₄ sur de l'acide thiosalicylique et mélange de ceux-ci ;
ii) ajouter goutte à goutte une solution de disulfure de 3,3-dihydroxy phényle obtenue par dissolution de disulfure de 3,3-dihydroxy phényle dans le benzène, sur ledit mélange acide thiosalicylique - H₂SO₄, conduisant ainsi à un mélange comprenant de l'acide thiosalicylique, H₂SO₄ et du disulfure de 3,3-dihydroxy phényle ;
iii) porter au reflux le mélange comprenant de l'acide thiosalicylique, H₂SO₄ et du disulfure de 3,3-dihydroxy phényle ;
iv) effectuer une réaction dans ledit mélange porté au reflux comprenant de l'acide thiosalicylique, H₂SO₄ et du disulfure de 3,3-dihydroxy phényle ;
v) terminer la réaction de l'étape (iv), conduisant à un mélange du produit ;
vi) filtrer, sécher, laver et purifier le mélange du produit obtenu à l'étape (v)

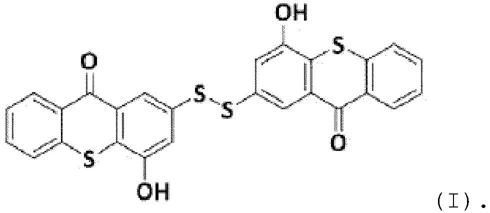

5. - Procédé selon la revendication 4, comprenant un ou plusieurs de ce qui suit :
m) avoir une concentration de l'acide thiosalicylique dans le mélange acide thiosalicylique - H₂SO₄ obtenu à l'étape (i) se situant dans la plage de 3,5×10⁻³ mole à 4,5×10⁻³ mole par 7 mL de H₂SO₄ ;
n) avoir une concentration de disulfure de 3,3-dihydroxy phényle dans la solution de disulfure de 3,3-dihydroxy phényle obtenue par dissolution de disulfure de 3,3-dihydroxy phényle dans le benzène à l'étape (ii) se situant dans la plage de 0,75×10⁻³ mole à 1,25×10⁻³ mole par 10 mL de benzène ;
o) appliquer le processus de reflux de l'étape (iii) pendant au moins 30 minutes à température ambiante, en faisant suivre par au moins 6 heures à une température dans la plage de 75 à 85°C ;
p) effectuer la fin de la réaction de l'étape (v) par précipitation du mélange porté au reflux comprenant de l'acide thiosalicylique, H₂SO₄ et du disulfure de 3,3-dihydroxyphényle, qui a réagi à l'étape (iv), dans de l'eau chaude ;
r) effectuer le processus de séchage de l'étape (vi) dans un four à vide ;
s) effectuer le processus de lavage de l'étape (vi) à l'aide d'éther de pétrole ;
t) conduire la purification de l'étape (vi) en présence d'une phase mobile éthanol : acétate d'éthyle, à l'aide d'un système à colonne.

6. - Procédé d'utilisation du composé [OH-TX-S-]₂ de Formule (I) dans la production de nanoparticules d'or (AuNps) par réduction photochimique, comprenant les étapes suivantes :
a) préparer un premier mélange contenant le composé [OH-TX-S-]₂ en solution conjointement avec un sel d'or ;
b) obtenir une première suspension contenant des AuNps en soumettant le premier mélange à un processus de réduction photochimique

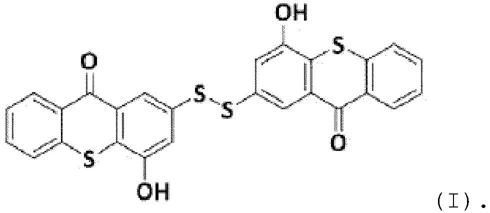

7. - Procédé selon la revendication 6, dans lequel le premier mélange contient, en tant que sel d'or, HAuCl₄ dissous dans un cosolvant puis dilué avec de l'eau à une concentration de 3 × 10⁻⁶ à 4 × 10⁻⁶ mol/litre, ainsi que [OH-TX-S-]₂ à une plage de concentration correspondant à 1/80 à 1/120 de ladite concentration.

8. - Procédé selon l'une quelconque des revendications 6 ou 7, dans lequel le processus de réduction photochimique est effectué en soumettant le premier mélange à un rayonnement correspondant à celui fourni par une lampe au mercure moyenne pression à 365 nm pendant plus de 300 secondes.

9. - Procédé selon l'une quelconque des revendications 6 à 8, comprenant une étape (c) de production d'une seconde suspension en mélangeant la première suspension avec une solution d'un médicament anticancéreux, après l'étape (b).

10. - Procédé selon la revendication 8, dans lequel ledit médicament anticancéreux est la doxorubicine.

11. - Procédé selon la revendication 10, dans lequel la concentration de doxorubicine dans la seconde suspension est dans la plage de 1,85 × 10⁻⁹ mol/litre à 9,17 × 10⁻⁷ mol/litre.

12. - Procédé selon la revendication 11, dans lequel la concentration de doxorubicine dans la seconde suspension est dans la plage de 1,85 × 10⁻⁹ mol/litre à 1,82 × 10⁻⁸ mol/litre.
